Europäisches Patentamt

(19) European Patent Office  (11) Numéro de publication: **0 048 010**

Office européen des brevets  **B1**

(12)  FASCICULE DE BREVET EUROPEEN

(45) Date de publication du fascicule du brevet: **10.04.85**  (51) Int. Cl.⁴: **A 41 B 13/02**

(21) Numéro de dépôt: **81107223.0**

(22) Date de dépôt: **14.09.81**

(54)  Procédé de fabrication de couches-culottes à jeter et couches-culottes obtenues par la mise en oeuvre de ce procédé.

(30) Priorité: **15.09.80 FR 8019862**

(43) Date de publication de la demande:
**24.03.82 Bulletin 82/12**

(45) Mention de la délivrance du brevet:
**10.04.85 Bulletin 85/15**

(84) Etats contractants désignés:
**AT BE CH DE GB IT LI**

(56) Documents cités:
**EP-A-0 023 804**
**DE-A-2 649 948**
**FR-A-2 082 803**
**FR-A-2 086 605**
**FR-A-2 177 425**

(73) Titulaire: **BOUSSAC SAINT FRERES B.S.F.**
**Société anonyme dite:**
**12, rue du Vieux Faubourg**
**F-59800 Lille (FR)**

(72) Inventeur: **de Jonckheere, Raphael**
**797, Domaine de la Vigne**
**F-59910 Bondues (FR)**
Inventeur: **Dussaud, Jacques**
**92, rue Jacquemars Gielée**
**F-59800 Lille (FR)**

(74) Mandataire: **Casalonga, Axel et al**
**BUREAU D.A. CASALONGA OFFICE JOSSE &**
**PETIT Baaderstrasse 12-14**
**D-8000 München 5 (DE)**

Courier Press, Leamington Spa, England.

## Description

La présente invention a pour objet un procédé de fabrication de couches-culottes à jeter présentant des découpes pour le passage des jambes de forme curviligne et munies sur au moins une partie leur pourtour interne d'éléments élastiques améliorant l'étanchéité au passage des jambes.

L'invention a également pour objet les couches-culottes obtenues par la mise en oeuvre du procédé de l'invention. Ces couches-culottes peuvent être jetées après usage et peuvent être utilisées pour les enfants en bas âge ou pour les personnes adultes incontinentes. Elles comportent une feuille imperméable externe, un coussin absorbant faisant partie intégrante de l'ensemble et de préférence un voile perméable formant revêtement interne de la couche-culotte.

Pour améliorer l'étanchéité au passage des jambes des couches-culottes à jeter de ce type, on a déjà préconisé de coller à l'état étiré des bandelettes élastiques rectilignes sur une feuille imperméable destinée à réaliser l'enveloppe extérieure de la couche-culotte.

A titre d'exemple de produits de ce type on peut citer les couches-culottes décrites dans les brevets américains US—A—3 860 003 et 4 050 462. Les éléments élastiques disposés au voisinage du pourtour des passages des jambes dans de telles couches-culottes qui ont pour but d'améliorer l'étanchéité à cet endroit, ne doivent pas cependant exercer un serrage trop important ou entraîner la formation de replis du matériau et notamment du matériau constituant le coussin absorbant afin de respecter l'anatomie de l'utilisateur qu'il s'agisse d'une personne adulte ou d'un enfant en bas âge.

On a déjà prévu pour améliorer le profil de couches-culottes équipées de tels élastiques d'entrejambe, une structure particulière du coussin absorbant tel que décrit dans la demande de brevet français FR—A—2 438 434.

La réalisation d'ouvertures ou de découpes d'entrejambe de profil curviligne ne permettait pas jusqu'à présent de fabriquer en continu de manière industrielle et à grande cadence des couche-culottes de ce type présentant des élastiques sur leur pourtour compte tenu de la forme incurvée du pourtour.

Des couches-culottes à jeter de ce type présentant un ruban élastique sur le bord du contour curviligne du passage des jambes tel que décrit dans le brevet français FR—A—2 063 794 ne peuvent être réalisées que par des moyens se prêtant peu à une fabrication à grande cadence et notamment par couture de fils ou de rubans élastiques.

La présente invention a pour objet un procédé de fabrication qui permet de réaliser de façon simple et à grande cadence des couches-culottes à jeter de ce type comportant des éléments élastiques bordant le contour curviligne de passages de jambes améliorant ainsi l'étanchéité à cet endroit tout en conservant au passage des jambes une forme proche de l'anatomie du l'utilisateur.

Le procédé de fabrication en continu suivant l'invention permet la réalisation de couches-culottes à jeter comprenant un coussin absorbant disposé entre une enveloppe extérieure imperméable à l'humidité et un voile intérieur perméable, l'ensemble comportant des découpes de profil curviligne pour permettre le passage de jambes, ces découpes étant munies sur le pourtour interne d'éléments élastiques. On dépose par intermittence un matériau adhésif à température elevée en fusion (hot melt) sur la face d'une bandelette élastique se déplaçant à l'état tendu et destiné à entrer en contact avec une feuille souple continue, d'un matériau thermosoudable imperméable à l'humidité. On déplace ensuite périodiquement dans une direction transversale au déplacement de ladite feuille imperméable, le point de contact entre la bandelette élastique encollée et la feuille imperméable en mouvement de façon à obtenir un élastique d'entrejambe de forme incurvée, collé à l'état tendu le long d'au moins une portion courbe de chaque découpe de passage de jambes. On fait s'enrouler la feuille imperméable sur au moins une portion de la périphérie d'un tambour rotatif à surface lisse dont la surface est maintenue à une température relativement basse par rapport à la température de l'adhésif en fusion, la différence de température étant d'au moins 20°C et de préférence entre environ 70°C et 130°C. De cette manière le matériau adhésif déposé à chaud sur la bandelette élastique étirée à l'état tendu avant le contact avec la feuille imperméable, subit un refroidissement brutal dès son contact avec ladite feuille imperméable ce qui entraîne un collage immédiat autorisant la réalisation d'un élastique curviligne collé à l'état étiré.

Le choix de la température du tambour rotatif peut aisément être opéré par le technicien en tenant compte des paramètres que constituent la masse dudit tambour, la masse de l'adhesif par unité de longueur de la bandelette élastique, la température de l'adhésif immédiatement avant le contact, la nature de l'adhésif et la courbure désirée de l'élastique d'entrejambe.

Il convient à cet égard que la différence de température entre l'adhésif déposé et la surface extérieure du tambour rotatif soit suffisante pour entraîner un effet de collage immédiat apte à résister aux efforts transversaux auxquels la bandelette est soumise étant donné le déplacement du point de contact.

Le point de contact entre la bandelette élastique et la feuille imperméable souple est de préférence maintenu fixe par intermittence de façon à définir des portions rectilignes non collées de bandelette élastique séparant les portions curvilignes collées.

Le déplacement en direction transversale du point de contact initial entre la bandelette élastique et la feuille imperméable souple peut se faire par tout moyen approprié. Dans un mode de réalisation préféré, on fait passer la bandelette élastique étirée sur un moyen de guidage tel qu'une glissière éventuellement incurvée ou une poulie à gorge montée à proximité immédiate de la périphérie du tambour rotatif, sur un chariot susceptible de se déplacer parallèlement à l'axe du tambour selon un mouvement alternatif de translation, la bandelette passant directement du moyen de guidage au point de contact avec la feuille souple imperméable en contact avec le tambour. Le moyen de guidage peut comporter des moyens de maintien de la bandelette tels que rebords ou orifices d'aspiration afin d'éviter que la bandelette élastique ne s'échappe du moyen de guidage lors de son mouvement. Ce mouvement peut être commandé par exemple par un dispositif de cames et indexé sur la rotation du tambour de façon que les portions curvilignes des bandelettes élastiques soient réalisées aux endroits appropriés sur la feuille imperméable.

Le procédé de l'invention peut être appliqué à la réalisation de couches-culottes obtenues par découpe transversale de la feuille imperméable continue. Dans ce cas, les coussins absorbants de forme allongée sont disposés selon l'axe longitudinal de la feuille imperméable et les découpes sont pratiquées sur les bords longitudinaux de la bande composite formée par la feuille imperméable, le coussin absorbant et le voile intérieur perméable.

Le procédé de l'invention peut également être appliqué à la réalisation de couches-culottes comportant un lien, de préférence élastique et continu, disposé à la taille. Dans ce cas, les coussins absorbants de forme allongée sont disposés perpendiculairement à l'axe longitudinal de la feuille imperméable et les découpes de passage des jambes sont pratiquées sous la forme d'ouvertures alignées selon l'axe longitudinal et symétriques par rapport à une direction transversale. Les couches-culottes sont alors obtenues par pliage ultérieur selon l'axe longitudinal de la bande composite suivi d'une découpe accompagnée d'une soudure simultanée selon l'axe transversal de symétrie des ouvertures.

La présente invention a également pour objet la couche culotte à jeter obtenu selon le procédé de l'invention.

Une telle couche-culotte à jeter comprend un coussin absorbant disposé entre une enveloppe extérieure imperméable à l'humidité et un voile intérieur perméable, l'ensemble comportant des découpes de profil curviligne pour permettre le passage des jambes, munies sur leur pourtour interne d'éléments élastiques. Selon l'invention, la couche-culotte fabriquée selon le procédé de l'invention comporte des élastiques d'entrejambe curvilignes collés le long d'au moins une portion courbe de chaque découpe de passage de jambes.

La présente invention sera mieux comprise à l'étude de la description de quelques modes de réalisation pris à titre d'exemples nullement limitatifs et illustrés par les dessins annexés sur lesquels:

La figure 1 est une vue schématique de côté des principaux éléments d'une installation permettant la mise en oeuvre du procédé de l'invention.

La figure 2 est une vue de dessus des éléments de la figure 1.

La figure 3 est une vue partielle d'une bande composite illustrant diverses étapes de fabrication d'une couche-culotte selon l'invention.

La figure 4 est une vue en élévation d'une couche-culotte fabriquée à partir de la bande composite de la figure 3.

La figure 5 est une bande composite illustrant la fabrication selon une variante de l'invention.

Et la figure 6 montre le stade final de fabrication dans la variante de la figure 5.

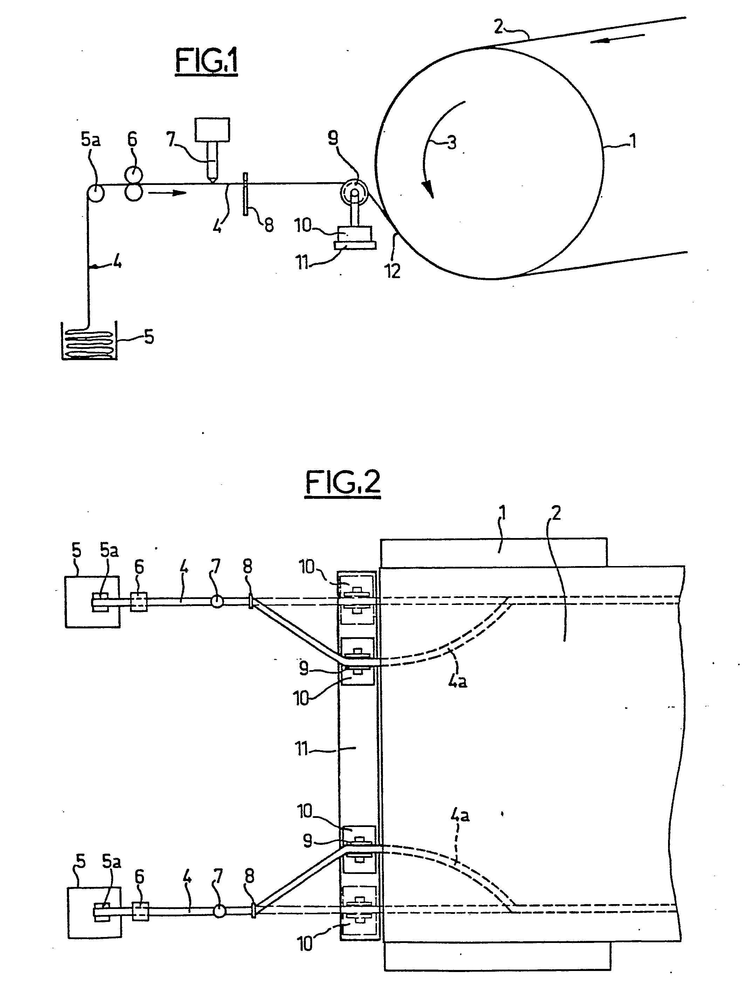

Telle quelle est représentée de manière très schématique sur les figures 1 et 2, l'installation premettant la mise en oeuvre du procédé de l'invention comprend un tambour rotatif 1 sur lequel vient s'enrouler une feuille souple continue 2 d'un matériau thermo-soudable imperméable à l'humidité tel que du polyéthylène. Le tambour 1 est entraîné en rotation dans le sens de la flèche 3 de sorte que la feuille 2 épouse sur environ la moitié de sa périphérie le tambour rotatif 1. Le tambour 1 est maintenu à une température relativement basse de l'ordre de 20°C. Le maintien de cette température peut être obtenu par un refroidissement artificiel forcé à l'intérieur du tambour ou simplement grâce à l'utilisation d'un tambour de masse suffisante.

Deux bandelettes élastiques 4 de faible largeur réalisées par exemple en latex sont stockées dans des conteneurs 5. Les bandelettes élastiques 4 passent après extraction des conteneurs 5 sur un galet de guidage 5a puis entre deux rouleaux tendeurs 6 entraînés en rotation de façon que la vitesse de déplacement linéaire de la bandelette élastique 4 à la sortie des rouleaux tendeurs 6 soit inférieure à la vitesse périphérique de la feuille imperméable 2 sur le tambour rotatif 1.

En réglant la différence de vitesse de manière convenable, on obtient l'élongation désirée de la bandelette élastique 4.

Un matériau adhésif à haute température par exemple de l'ordre de 100°C à 150°C, rendu liquide par fusion (hot melt) est délivré de manière intermittente par la buse 7 sous forme d'une ligne fine sur la surface de la bandelette élastique 4 destinée à entrer en contact avec la feuille imperméable 2. Une valve non représentée sur la figure permet d'arrêter de manière automatique pendant des périodes de temps déterminées l'alimentation en matériau adhésif. La bandelette élastique 4 éventuellement revêtue d'une ligne de matériau adhésif passe ensuite à l'intérieur d'un organe de guidage 8 fixe puis sur une poulie à gorge 9 montée sur un chariot mobile 10 pouvant coulisser sur une glissière 11 sous l'action d'organes de commande non représentés sur les figures qui peuvent par exemple comporter des cames ou des organes analogues. Après son passage sur la poulie à gorge 9, la bandelette élastique 4 vient en contact avec la feuille imperméable 2 à la périphérie du tambour rotatif 3, le point de contact étant référencé 12 sur les figures. La poulie à gorge 9 est disposée à proximité immédiate de la périphérie du tambour rotatif 3.

Dans l'exemple illustré le point de contact 12 se trouve légèrement en aval de la moitié du parcours périphérique de la feuille imperméable 2 sur le tambour rotatif. On pourrait également disposer le point de contact plus en amont du trajet de la feuille 2 de façon à augmenter la durée de refroidissement de l'adhésif en contact avec le tambour 3. La bandelette élastique 4 se dirige depuis la poulie à gorge 9 jusqu'au contact avec la feuille imperméable 2 se trouvant à la périphérie du tambour rotatif 3 selon une direction tangente par rapport au profil du tambour rotatif 3.

Dans ces conditions et compte tenu de l'écart de température entre le tambour et l'adhésif, le matériau adhésif en fusion se trouvant à la surface de la bandelette élastique 4 se fige aussitôt après le contact entrainant un collage immédiat de sorte que le déplacement en direction transversale des chariots 10 et des points de contact 12, permet le collage de la bandelette élastique selon un profil curviligne 4a comme on peut le voir sur la figure 2. L'écart de température, compte tenu de la masse respective de l'adhésif en fine ligne d'encollage et du tambour rotatif est suffisant pour que l'adhésif se fige dès son contact et résiste à l'effort transversal dû à la pose de la bandelette selon un trajet incurvé.

Sur la figure 2, on a représenté également en tirets la position occupée par les chariots 10 pour la pose des portions rectilignes des bandelettes élastiques. On remarquera sur la figure 2 que l'angle formé par la bandelette élastique à la sortie de l'organe de guidage 8 a été exagéré pour la clarté du dessin. Dans la pratique on fait en sorte que cet angle maximal ne risque pas d'entraîner l'extraction de la bandelette 4 des poulies à gorge 9. Dans une variante les poulies rotatives 9 peuvent être remplacées par des glissières ou autres moyens de guidage et de maintien de la bandelette élastique en déplacement.

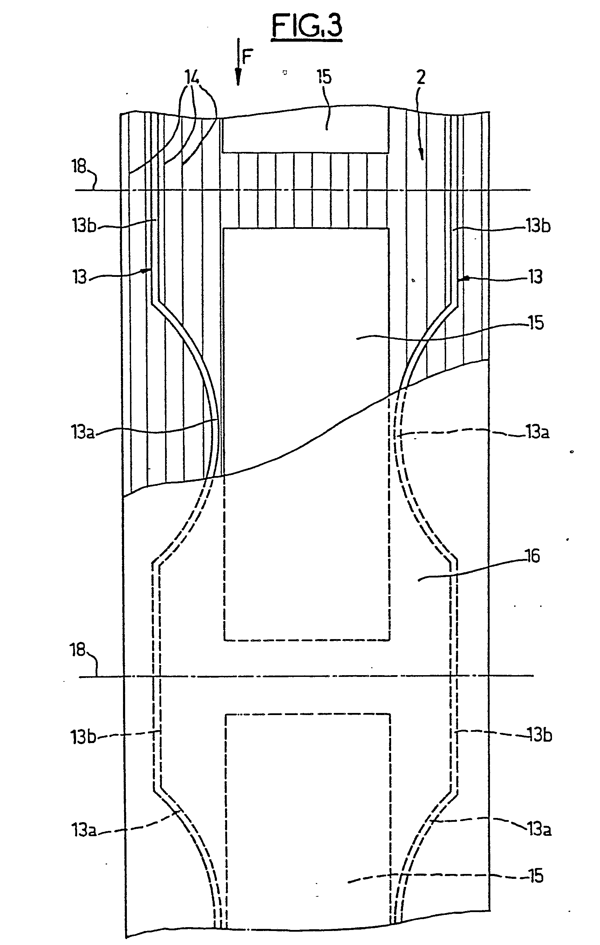

Dans le mode de réalisation illustré sur la figure 3, la feuille imperméable 2, qui se déroule dans le sens de la flèche F parallèlement à l'axe longitudinal de la bande 2, reçoit le long de ses deux bords longitudinaux deux élastiques continus 13. Les portions curvilignes concaves 13a sont encollées à l'état étiré selon le procédé qui a été décrit en référence aux figures 1 et 2 tandis que les portions rectilignes 13b qui les relient entre elles ne sont pas encollées. Des lignes continues d'adhésif 14 déposées sur la surface de la feuille imperméable 2 dans une autre étape du procédé permettent le collage de coussins absorbants 15 de forme allongée rectangulaire et d'un voile perméable à l'humidité 16.

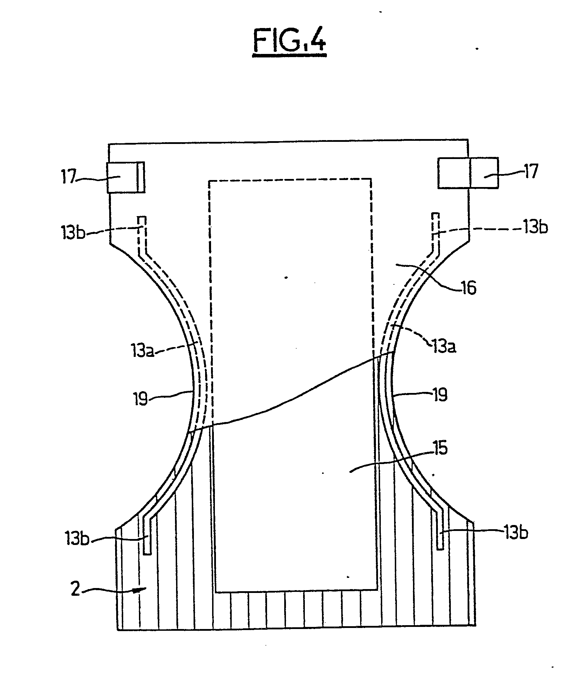

Après la pose de dispositifs de rubans adhésifs 17 sur les bords longitudinaux de la feuille 2 et découpe incurvée et soudure simultanée pratiquées sur les bords longitudinaux de la bande composite formée par la feuille 2, les coussins absorbants 15 et le voile perméable 16, on procède au stade final de fabrication consistant à découper transversalement selon les lignes 18 la bande composite obtenue de façon à former les couches-culottes telles que celle qui est illustrée sur la figure 4. Lors de la découpe, les portions rectilignes 13b des élastiques d'entrejambe 13 sont sectionnées, les portions subsistantes reprenant leur longueur initiale et restant emprisonnées entre la feuille imperméable 2 et le voile perméable 16.

On obtient finalement comme on peut le voir sur la figure 4, une couche-culotte dans laquelle le passage des jambes 19 présente un profil incurvé concave s'adaptant parfaitement à l'anatomie de l'utilisateur, le profil 19 étant bordé intérieurement d'un élastique curviligne 13a posé à l'état étiré et assurant une étanchéité convenable à cet endroit.

Bien que le coussin absorbant ait été représenté sur la figure 4 comme présentant une forme rectangulaire on comprendra qu'il pourrait également présenter une forme de sablier suivant le profil des ouvertures de passage de jambes 19.

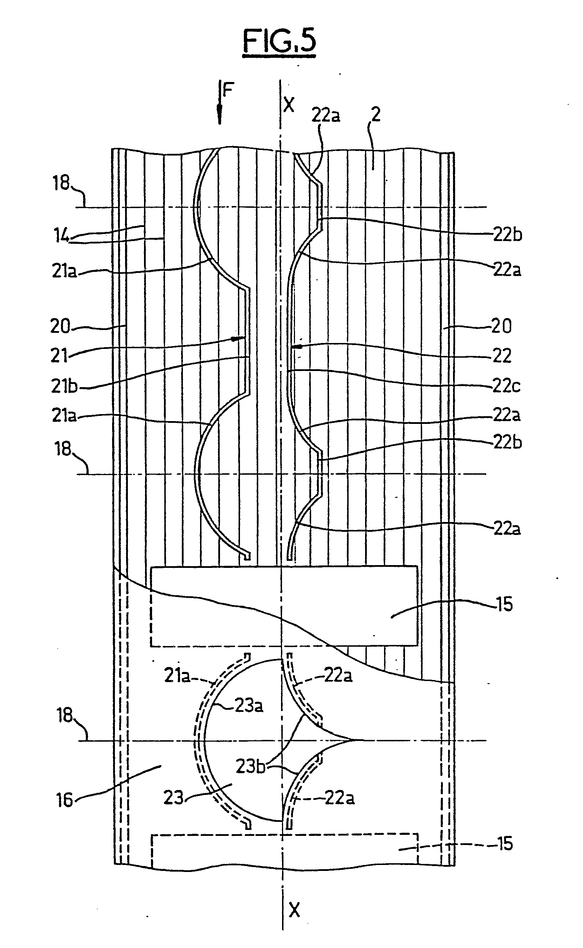

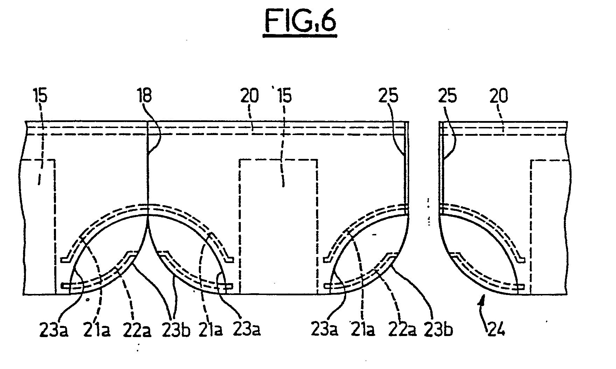

Dans le mode de réalisation illustré dans les figures 5 et 6 où les éléments identiques portent les mêmes références, la feuille imperméable 2 se déplaçant comme précédemment selon la flèche F parallèlement à l'axe longitudinal X—X reçoit tout d'abord tout le long de ses deux bords longitudinaux un élastique continu 20 rectiligne encollé à l'état étiré sur toute sa surface et destiné à former un élastique continu de serrage à la taille de la couche-culotte. Les élastiques d'entrejambe sont constitués par deux bandelettes élastiques 21 et 22 encollées de manière intermittente respectivement d'un côté et de l'autre de l'axe longitudinal X—X. La bandelette élastique 21 comporte des portions curvilignes 21a par rapport à la future ouverture de passage de jambe et en forme de demicercle symétriques par rapport aux lignes transversales 18 et coupant ces dernières perpendiculairement. Ces portions curvilignes 21a encollées sur toute leur longueur obtenues par le procédé illustré sur les figures 1 et 2 sont reliées entre elles par des portions rectilignes non encollées 21b parallèle à l'axe longitudinal X—X.

Les élastiques 22 comportent deux portions curvilignes 22a encollées sur toute leur longueur disposées de chaque côté de la ligne transversale 18 symétriquement par rapport à cette dernière en forme d'arc de cercle de profil convexe par rapport à la future ouverture de passage de jambe. Les portions encollées 22a sont reliées par une portion rectiligne 22b non encollée coupant la ligne transversale 18 et parallèle à l'axe X—X et une deuxième portion rectiligne non encollée 22c parallèle à l'axe X—X au voisinage immédiat de ce dernier.

Lors d'une opération ultérieure, les portions rectilignes non encollées 21b et 22c sont sectionnées et retirées ou simplement relachées de façon à permettre la pose des coussins absorbants 15 de forme allongée rectangulaire qui sont cette fois disposés de manière transversale c'est-à-dire perpendiculairement à l'axe longitudinal X—X entre deux lignes transversales 18. Le collage des matelas absorbants 15 se fait comme précédemment grâce à l'existence des lignes d'encollage 14.

Après la pose et le collage du voile perméable 16, on procède au découpage et au soudage simultanés sur leur pourtour des ouvertures de passage de jambe 23 dont le profil comporte un premier arc de cercle 23a concave parallèle à l'arc de cercle de la portion 21a de l'élastique 21 et deux portions d'arc de cercles convexes 23b parallèles respectivement aux arcs de cercles 22a de l'élastique 22. La pointe formée par les deux arcs de cercle 23b qui se rejoignent sensiblement tangentiellement sur la ligne transversale 18 entraine en même temps le découpage de la portion rectiligne non encollée 22b de l'élastique 22.

La fabrication se termine par un pliage autour de l'axe longitudinal X—X de façon à obtenir le produit continu représenté sur la partie gauche de la figure 6.

Une découpe accompagnée d'une soudure simultanée permet la réalisation des couches-culottes telles que la couche-culotte 24 qui comporte une soudure 25 sur son côté latéral. La couche-culotte obtenue s'apparente donc à un slip et s'enfile comme tel, l'élastique de taille 20 étant rendu continu par les deux soudures latérales 25.

Dans tous les cas, le procédé de l'invention autorisant la pose à l'état tendu d'éléments élastiques de forme curviligne permet donc la réalisation d'élastiques d'entrejambe suivant le profil curviligne de l'ouverture de passage de jambe la mieux adaptée à l'anatomie de l'utilisateur.

## Revendications

1. Procédé de fabrication en continu de couches-culottes à jeter comprenant un coussin absorbant (15) posé entre une enveloppe extérieure imperméable à l'humidité (2) et un voile intérieur perméable (16), l'ensemble comportant des découpes de profil curviligne (19) pour permettre le passage des jambes, munies sur leur pourtour interne d'éléments élastiques (13), procédé dans lequel on dépose par intermittence un matériau adhésif à température élevée sur la face d'une bandelette élastique (4) se déplaçant à l'état tendu et destinée à entrer en contact avec une feuille souple continue (2) d'un matériau thermosoudable imperméable à l'humidité et on déplace périodiquement dans une direction transversale au déplacement de ladite feuille imperméable (2) le point de contact (12) entre la bandelette élastique encollée (4) et la feuille imperméable (2) en mouvement de façon à obtenir un élastique d'entrejambe de forme incurvée collé le long d'au moins une portion courbe de chaque découpe de passage de jambes, caractérisé par le fait que l'on fait s'enrouler la feuille souple imperméable (2) sur une portion au moins de la périphérie d'un tambour rotatif (1) à surface lisse dont on maintient la surface à une température inférieure à celle de l'adhesif, la différence de température étant d'au moins 20°C et de préférence entre environ 70°C et 130°C afin que l'adhésif soit capable, dès son contact, de résister aux efforts transversaux dûs à la pose de l'élastique étiré à l'état tendu sur la feuille souple imperméable en contact avec ledit tambour, selon un profil incurvé.

2. Procédé de fabrication selon la revendication 1 caractérisé par le fait que le point de contact précité (12) est maintenu fixe par intermittence de façon à définir de portions rectilignes (13b) non collées des bandelettes élastiques séparant les portions curvilignes collées (13a).

3. Procédé de fabrication selon l'une des revendications 1 ou 2 caractérisé par le fait que le point de contact (12) se trouve légèrement en aval de la moitié du parcours périphérique de la feuille imperméable (2) sur le tambour (1).

4. Procédé de fabrication selon l'une quelconque des revendications 1 à 3, caractérisé par le fait que l'on fait passer la bandelette élastique étirée (4) sur un moyen de guidage (9) monté à proximité immédiate de la périphérie du tambour rotatif (1) sur un chariot (10) susceptible de se déplacer parallèlement à l'axe du tambour (1) selon un mouvement alternatif de translation, la bandelette (4) passant directement du moyen de guidage (9) au point de contact (12) avec la feuille souple imperméable (2) en contact avec ledit tambour (1).

5. Procédé selon la revendication 4 caractérisé par le fait que le moyen de guidage comporte des moyens de maintien de la bandelette afin d'éviter qu'elle ne s'échappe lors de son mouvement.

6. Procédé selon les revendications 4 ou 5, caractérisé par le fait que le mouvement du moyen de guidage est commandé en fonction de la rotation du tambour.

7. Procédé de fabrication selon l'une quelconque des revendications précédentes caractérisé par le fait que les coussins absorbants de forme allongée (15) sont disposés selon l'axe longitudinal de la feuille imperméable (2) et que des découpes sont pratiquées sur les bords longitudinaux de la bande composite formée par la feuille imperméable, les coussins absorbants et le voile intérieur, les couches-culottes étant finalement obtenues par découpe transversale.

8. Procédé de fabrication selon l'une quelconque des revendications 1 à 6, caractérisé par le fait que des coussins absorbants de forme allongée (15) sont disposés perpendiculairement à l'axe longitudinal de la feuille imperméable et que des découpes sont pratiquées sous forme d'ouvertures (22) selon l'axe longitudinal et symétriquement par rapport à une direction transversale, les couches-culottes étant finalement obtenues par pliage selon l'axe longitudinal suivi d'une découpe et d'une soudure selon l'axe transversal de symétrie (18) des ouvertures.

9. Procédé de fabrication selon la revendication 8, caractérisé par le fait qu'un élastique continu rectiligne (20) est collé à l'état étiré sur toute la longueur de chaque bord longitudinal de la feuille imperméable de façon à définir un serrage élastique à la taille de la couche-culotte.

10. Couche-culotte à jeter comprenant un coussin absorbant (15) disposé entre une enveloppe extérieure (2) imperméable à l'humidité et un voile intérieur perméable (16), l'ensemble comportant des découpes (19, 23) de profil curviligne pour permettre le passage du jambe, munies sur leur pourtour interne d'éléments élastiques (13, 21, 22), caractérisé par le fait qu'elle a été fabriquée selon le procédé de l'une quelconque des revendications précédentes et qu'elle comporte des élastiques d'entrejambe curvilignes collés le long d'au moins une portion courbe de chaque découpe de passage de jambe.

**Patentansprüche**

1. Verfahren zur kontinuierlichen Herstellung von Wegwerfwindeln, die ein saugfähiges Kissen (15) umfassen, das zwischen einer feuchtigkeitsundurchlässigen Außenhülle (2) und einem durchlässigen Innentuch (16) angeordnet ist, wobei die Anordnung Ausschnitte mit gekrümmtem Profil (19) aufweist, um den Durchtritt der Beine zu ermöglichen und die längs ihres inneren Außenumfanges mit elastischen Elementen (13) ausgestattet sind, bei welchem Verfahren man bei erhöhter Temperatur mit Unterbrechungen einen Klebstoff auf die eine Seite eines elastischen Bändchens (4) aufbringt, das im gespannten Zustand vorgeschoben wird und das dazu bestimmt ist, mit einer kontinuierlichen, weichen Folie (2) eines warm schweißbaren, feuchtigkeitsundurchlässigen Materials in Berührung zu treten und bei dem man in einer Richtung quer zur Vorschieberichtung der erwähnten undurchlässigen Folie (2) periodisch den Berührungspunkt (12) zwischen dem mit Klebstoff beschichteten, elastischen Bändchen (4) und der undurchlässigen Folie (2), die in Bewegung ist, verschiebt, um einen elastischen Schrittbereich mit nach innen gekrümmter Form zu erhalten, der entlang wenigstens eines gekrümmten Teils jedes Ausschnittes für den Durchtritt der Beine angeklebt ist, dadurch gekennzeichnet, daß man sich die weiche undurchlässige Folie (2) wenigstens um einen Teil der Umfangsfläche einer drehbaren Trommel mit glatter Oberfläche herumwickeln läßt, wobei man deren Oberfläche auf eine Temperatur unter jener des Klebstoffes hält, wobei der Temperaturunterschied wenigstens 20°C, vorzugsweise zwischen 70 und 130°C beträgt, so daß der Klebstoff gleich nach seiner Berührung Querkräften, die durch das Auflegen des in einem gespannten Zustand gedehnten, elastischen, auf die weiche, undurchlässige Folie, die mit dieser Trommel in Berührung steht, längs einer gekrümmten Linie aufgelegt ist, entstehen, widerstehen kann.

2. Verfahren zur Herstellung nach Anspruch 1, dadurch gekennzeichnet, daß der vorgenannte Berührungspunkt (12) mit Unterbrechungen ortsfest gehalten wird, so daß gerade, nicht geklebte Abschnitte (13b) des elastischen Bändchens die geklebten, gekrümmten Abschnitte (13a) voneinander trennen.

3. Verfahren zur Herstellung nach einem der Ansprüche 1 oder 2, dadurch gekennzeichnet, daß der Berührungspunkt (12) in Bewegungsrichtung etwas nach der Hälfte des Umfangsweges der undurchlässigen Folie (2) auf der Trommel (1) liegt.

4. Verfahren zur Herstellung nach irgendeinem der Ansprüche 1 bis 3, dadurch gekennzeichnet, daß man das gedehnte, elastische Bändchen (4) über eine Führung (9) bewegt, die in unmittelbarer Nachbarschaft des Umfanges der drehbaren Trommel (1) auf einem Schlitten (10) montiert ist, der parallel zur Achse der Trommel (1) in einer alternierenden Translationsbewegung verschiebbar ist, wobei sich das Bändchen (4) von der Führung (9) unmittelbar zum Berührungspunkt (12) mit der weichen, undurchlässigen Folie (2), die mit der Trommel (1) in Berührung steht, bewegt.

5. Verfahren nach Anspruch 4, dadurch gekennzeichnet, daß die Führung Haltemittel für das Bändchen aufweist, um zu vermeiden, daß dieses während seiner Bewegung abgleitet.

6. Verfahren nach den Ansprüchen 4 oder 5, dadurch gekennzeichnet, daß die Bewegung der Führung in Abhängigkeit von der Drehung der Trommel gesteuert ist.

7. Verfahren zur Herstellung nach irgendeinem der vorhergehenden Ansprüche, dadurch gekennzeichnet, daß die saugfähigen Kissen (15) mit länglicher Form in Richtung der Längsachse der undurchlässigen Folie (2) angeordnet werden und daß die Ausschnitte an den Längsrändern des durch die undurchlässige Folie, die saugfähigen Kissen und das Innentuch gebildeten, zusammengesetzten Bandes ausgeführt werden, wobei die Windeln schließlich durch einen Querschneidevorgang erhalten werden.

8. Verfahren zur Herstellung nach irgendeinem der Ansprüche 1 bis 6, dadurch gekennzeichnet, daß die saugfähigen Kissen (15) mit länglicher Form senkrecht zur Längsachse der undurchlässigen Folie angeordnet werden, und daß die Ausschnitte in Form von Öffnungen (22) entlang der Längsachse und symmetrisch, bezogen auf eine Querrichtung ausgeführt werden, wobei die Windeln schließlich durch Falten entlang der Längsachse, gefolgt von einem Schneidevorgang und einem Schweißvorgang, entlang der Quersymmetrieachse (18) der Ausschnitte erhalten werden.

9. Verfahren zur Herstellung nach Anspruch 8, dadurch gekennzeichnet, daß ein gerades, fortlaufendes Gummiband (20) im gedehnten Zustand über die gesamte Länge jedes Längsrandes der undurchlässigen Folie aufgeklebt wird, um ein elastisches Zusammenziehen in der Taille der Windel zu bilden.

10. Wegwerfwindel mit einem saugfähigen Kissen (15), das zwischen einer feuchtigkeitsundurchlässigen Außenhülle (2) und einem durchlässigen Innentuch (16) angeordnet ist, wobei die Anordnung Ausschnitte (19, 23) mit gekrümmtem Profil für den Durchtritt von Beinen aufweist, die längs ihres inneren Außenumfanges mit elastischen Elementen (13, 21, 22) ausgestattet sind, dadurch gekennzeichnet, daß sie gemäß dem Verfahren irgendeines der vorhergehenden Ansprüche hergestellt sind und daß sie im Zwickelbereich gekrümmte Gummibänder aufweist, die entlang wenigstens eines gebogenen Teils jedes Ausschnittes für den Durchtritt des Beins angeklebt sind.

## Claims

1. Process for continuous manufacture of disposable nappy-pants comprising an absorbent pad (15) placed between a moisture-proof outer cover (2) and a permeable inner voile (16), the whole incorporating cutouts with a curved outline (19) to enable legs to pass, which are provided with elastic elements (13) at their inner periphery, a process in which a material which is adhesive at elevated temperature is intermittently deposited on the face of an elastic strip (4) which moves in the stretched state and is intended to come into contact with a continuous supple sheet (2) of a moisture-proof heat-weldable material, and the point of contact (12) between the glue-coated elastic strip (4) and the moving impermeable sheet (2) is moved periodically in a direction transverse to the movement of the said impermeable sheet (2) so as to obtain a crotch-elastic band of curved shape glued along at least one curved portion of each leg-passage cutout, characterised in that the impermeable supple sheet (2) is wound on at least a part of the periphery of a smooth-surfaced rotary drum (1) the surface of which is kept at a temperature below that of the adhesive, the temperature difference being at least 20°C and preferably between approximately 70°C and 130°C in order that the adhesive be capable, as soon as contacted, of withstanding transverse forces due to the placing of the stretched elastic band in the taut state on the impermeable supple sheet in contact with the said drum, along a curved outline.

2. Manufacturing process according to Claim 1, characterised in that the abovementioned point of contact (12) is kept intermittently fixed so as to define the rectilinear non-glued portions (13b) of the elastic strips separating the glued curved portions (13a).

3. Manufacturing process according to either of Claims 1 or 2, characterised in that the point of contact (12) is slightly downstream of halfway of the peripheral path of the impermeable sheet (2) on the drum (1).

4. Manufacturing process according to any one of Claims 1 to 3, characterised in that the stretched elastic strip (4) is passed over a guiding means (9) fitted in the immediate vicinity of the periphery of the rotary drum (1) on a trolley (10) capable of moving parallel to the drum axis (1) with reciprocal translational motion, the strip (4) passing directly from the guiding means (9) to the point of contact (12) with the impermeable supple sheet (2) in contact with the said drum (1).

5. Process according to Claim 4, characterised in that the guiding means comprises means for holding the strip to prevent the latter from escaping when it moves.

6. Process according to Claims 4 or 5, characterised in that the motion of the guiding means is controlled as a function of the rotation of the drum.

7. Manufacturing process according to any one of the preceding claims, characterised in that the absorbent pads of elongated shape (15) are arranged along the longitudinal axis of the impermeable sheet (2) and cutouts are made on the longitudinal edges of the composite strip formed by the impermeable sheet, the absorbent pads and the inner voile, the nappy-pants being finally obtained by transverse cutting-out.

8. Manufacturing process according to any one of Claims 1 to 6, characterised in that absorbent pads of elongated shape (15) are arranged at right angles to the longitudinal axis of the impermeable sheet and cutouts are made in the shape of openings (22) along the longitudinal axis and symmetrically relative to a transverse direction, the nappy-pants being finally obtained by folding along the longitudinal axis, followed by cutting-out and welding along the transverse axis of symmetry (18) of the openings.

9. Manufacturing process according to Claim 8, characterised in that a continuous rectilinear elastic band (20) is glued in the stretched state over the whole length of each longitudinal edge of the impermeable sheet so as to define a resilient tightening at the waist of the nappy-pants.

10. Disposable nappy-pants incorporating an absorbent pad (15) arranged between a humidity-proof outer cover (2) and a permeable inner voile (16), the whole incorporating cutouts (19, 23) of curved outline to permit legs to pass, which are provided on their inner periphery with elastic elements (13, 21, 22), characterised in that it has been manufactured according to the process of any one of the preceding claims, and that it incorporates curved crotch elastic bands glued along at least one curved portion of each cutout leg-passage cutout.

## FIG.1

## FIG.2

# 0 048 010

## FIG.3

# FIG.4

# FIG.5

## FIG.6

15 18 20 '15 25 25 20

23a 21a 22a 23b 21a 23a  23a 21a 22a 23b 24